# EUROPÄISCHE PATENTSCHRIFT

(11) **EP 3 260 113 B1**
(45) Veröffentlichungstag und Bekanntmachung des Hinweises auf die Patenterteilung: **30.01.2019**
(21) Anmeldenummer: 17171692.1
(22) Anmeldetag: 18.05.2017
(51) Int. Cl.: A61K 8/37, A61Q 17/04

(54) **SONNENSCHUTZMITTEL MIT REDUZIERTER TEXTILVERFLECKUNG**
SUNSCREEN AGENT WITH REDUCED TEXTILE DISCOLOURATION
PROTECTION SOLAIRE AYANT UNE TENDANCE RÉDUITE À TÂCHER LES TISSUS

(30) Priorität: 23.06.2016 DE 102016211238
(43) Veröffentlichungstag der Anmeldung: 27.12.2017
(73) Patentinhaber: Beiersdorf AG, 20253 Hamburg (DE)
(72) Erfinder: Weinert, Katrin, 22763 Hamburg (DE); Borchers, Kathrin, 21614 Buxtehude (DE); Sprock, Sarah, 22297 Hamburg (DE); Eisert, Anja, 22085 Hamburg (DE); Bleckmann, Andreas, 22926 Ahrensburg (DE)

(56) Entgegenhaltungen:
- EP-A2- 2 570 109
- WO-A1-2015/165713
- WO-A1-2015/165715

## Beschreibung

Die vorliegende Erfindung betrifft ein Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche diese UV-A Filter und/oder Breitbandfilter enthält, kontaminiert sind, dadurch gekennzeichnet, dass der kosmetischen Zubereitung bei Raumtemperatur flüssige Öle in einer Menge zugesetzt werden, die größer ist als die Menge, welche notwendig ist, um diese UV-Filter bei Raumtemperatur in diesen Ölen zu lösen, sowie die Verwendung von bei Raumtemperatur flüssigen Ölen in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge. WO 2015/165715 löst dieses Problem durch den Zusatz von Komplexbildnern.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoy)methan und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche (d.h. öllösliche) UV-A und/oder Breitbandfilter Filter (insbesondere 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine)) zu entwickeln, welche sich leichter aus den mit der Zubereitung kontaminierten Textilen herauswaschen lassen.

Überraschend gelöst wird die Aufgabe durch die Verwendung von bei Raumtemperatur flüssigen Ölen in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, dadurch gekennzeichnet, dass die Mindestmenge an bei Raumtemperatur flüssigen Ölen (Gesamtmenge) im Verhältnis zur Gesamtmenge an organischen, öllöslichen UV-A Filtern und organischen, öllöslichen Breitbandfiltern, bezogen auf das Gewicht Faktor 3 beträgt.

Es wurde festgestellt, das sich die organischen, öllöslichen UV-A Filter und organischen, öllöslichen Breitbandfilter (insbesondere 4-(tert.-Butyl)-4'-methoxydibenzoylmethan, (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine)) umso leichter aus Textilien wieder auswaschen lassen, je höher die Einsatzkonzentration an bei Raumtemperatur flüssigen Ölen in der kosmetischen Zubereitung ist, welche diese UV-Filter enthält und mit der das Textil verunreinigt (kontaminiert) wurde. Dieser Sachverhalt war umso überraschender, weil lipophile Stoffe schnell in Gewebe eindringen und sich grundsätzlich relativ schlecht aus Textilien auswaschen lassen. Es war vielmehr erwartet worden, dass ein höherer Anteil an Ölen dazu führen würde, dass die UV-Filter tiefer in das Textilgewebe eindringen (und damit schwerer zu entfernen sein) würden.

Erfindungsgemäß ist die Raumtemperatur definiert als 20 °C. Alle Angaben zum Aggregatszustand beziehen sich darüber hinaus auf einen Druck von 1,013 bar.

Erfindungsgemäß vorteilhafte Ausführungsformen der erfindungsgemäßen Verwendung sind dadurch gekennzeichnet, dass es sich bei den organischen, öllöslichen UV-A Filtern und/oder organischen, öllösichen Breitbandfiltern um wie 4-(tert.-Butyl)-4'-methoxydiben-zoylmethan, (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) handelt.

Es ist erfindungsgemäß bevorzugt für die erfindungsgemäße Verwendung, wenn es sich bei den bei Raumtemperatur flüssigen Ölen um n-Dibutyladipat, C12-15 Alkyl Benzoate, Butylene Glycol Dicaprylate/Dicaprate, Ethylhexylsalicylat, (Octylsalicylate), Homomenthylsalicylat (Homosalate), Ethylhexyl-2-cyano-3,3-diphenylacrylat (Octocrylen) und deren Mischungen handelt.

Enthält die kosmetische Zubereitung eine Kombination aus 4-(tert.-Butyl)-4'-methoxydiben-zoylmethan und/ oder Bis-Ethylhexyloxyphenol Methoxyphenyl Triazine und/ oder Diethylamino Hydroxybenzoyl Hexyl Benzoate, so ist es für die erfindungsgemäße Verwendung bevorzugt, wenn als bei Raumtemperatur flüssige Öle die folgenden Verbindungen eingesetzt werden: n-Dibutyladipat, C12-15 Alkyl Benzoate, Butylene Glycol Dicaprylate/Dicaprate, Ethylhexylsalicylat, (Octylsalicylate), Homomenthylsalicylat (Homosalate), Ethylhexyl-2-cyano-3,3-diphenylacrylat (Octocrylen) und deren Mischungen. In diesem Falle ist es erfindungsgemäß bevorzugt, wenn das Gewichtsverhältnis der Summe der öllöslichen UV-A Filtern und organischen, öllöslichen Breitbandfiltern zur Gesamtmenge der bevorzugt verwendeten Öle mindestens Faktor 1:3 ist.

Es ist erfindungsgemäß vorteilhaft, wenn bei der erfindungsgemäßen Verwendung in den Zubereitungen die Einsatzkonzentration an4-(tert.-Butyl)-4'-methoxydibenzoylmethan von 0,01 bis 5,0 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an2,4-Bis-{[4-(2-ethyl-hexyloxy)-2-hydroxy]-phenyl)-6-(4-methoxyphenyl)-1,3,5-triazin (INCI Bis-Ethylhexyloxyphenol Methoxyphenyl Triazine) von 0,01 bis 10 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an (2-[-4-(Diethylamino)-2-hydroxybenzoyl] Benzoesäurehexylester (INCI: Diethylamino Hydroxybenzoyl Hexyl Benzoate) von 0,01 bis 10 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an n-Dibutyladipat von 0,01 bis 10 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an C12-15

Alkyl Benzoate von 0,01 bis 15 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an Butylene Glycol Dicaprylate/Dicaprate von 0,01 bis 15 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an Ethylhexylsalicylat, (Octylsalicylate) von 0,01 bis 5 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an Homomenthylsalicylat (Homosalate) von 0,01 bis 10 Gewichts-%, bezogen auf das Gesamtgewicht der Zubereitung, beträgt.

Es ist erfindungsgemäß vorteilhaft, wenn bei der erfindungsgemäßen Verwendung in den Zubereitungen die Einsatzkonzentration an Ethylhexyl-2-cyano-3,3-diphenylacrylat (Octocrylen) von 0 bis 10 Gewichts-%, bezogen auf das Gesamtgewicht der Zubereitung, beträgt.

Darüber hinaus kann die kosmetische Zubereitung vorteilhaft noch weitere UV-Fiiter enthalten, die beispielsweise gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-borny-lidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid.

Dabei ist es erfindungsgemäß von Vorteil, wenn die erfindungsgemäße Zubereitung frei ist von 2-Ethylhexyl-2-cyano-3,3-diphenylacrylat, 2-Hydroxy-4-methoxybenzophenon und/oder 3-(4-Methylbenzyliden)campher. Dabei sollte bevorzugt auf den Einsatz von 3-(4-Methylbenzyliden)campher und 2-Hydroxy-4-methoxybenzophenon verzichtet werden.

Der erfindungsgemäße Effekt kann dadurch erfindungsgemäß verbessert werden, dass man dem Kosmetikum ein oder mehrere Komplexbildner zusetzt.

Dabei ist es erfindungsgemäß bevorzugt, wenn als Komplexbildner eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat (IDS)
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
   und/oder deren Alkalisalze eingesetzt werden.

Es ist erfindungsgemäß besonders bevorzugt, wenn als Komplexbildner eine oder mehrere der Verbindungen aus der Gruppe
- Diethylentriaminpenta(methylenphosphonsäure)/ DTPMP
- Ethylendiamintetra(methylenphosphonsäure/ EDTMP
- Aminotrimethylenphosphonsäure/ ATMP
- Phosphonobutan-tricarbonsäure/ PBTC
- Iminodisuccinat (IDS)
- Natriumpolyphosphat
- Tetranatriumpyrophosphat
- Bernsteinsäure
   und/oder deren Alkalisalze und/ oder deren Amin-N-Oxide eingesetzt werden.

Die erfindungsgemäß bevorzugte Gesamt-Einsatzkonzentration für die Komplexbildner (einer oder mehrere) beträgt dabei von 0,01 bis 3 Gewichts-%, bezogen auf das Gesamtgewicht der Zubereitung.

Der erfindungsgemäße Effekt kann dadurch erfindungsgemäß verbessert werden, dass man dem Kosmetikum 1-Hydroxy-4-methyl-6-(2,4,4-trimethylpentyl)-2(1H)-pyridon (Pirocton) und/oder dessen Monoethanolamin-Salz zugesetzt wird.

Die erfindungsgemäß bevorzugte Gesamt-Einsatzkonzentration für Pirocton beträgt dabei von 0,01 bis 1 Gewichts-%, bezogen auf das Gesamtgewicht der Zubereitung.

Der erfindungsgemäße Effekt kann dadurch erfindungsgemäß verbessert werden, dass man dem Kosmetikum Siloxanelastomere zusetzt.

Die erfindungsgemäß bevorzugte Gesamt-Einsatzkonzentration für Siloxanelastomere (einer oder mehrere) beträgt dabei von 0,1 bis 20 Gewichts-%, bezogen auf das Gesamtgewicht der Zubereitung.

Nicht zuletzt kann der erfindungsgemäße Effekt dadurch erfindungsgemäß verbessert werden, dass man dem Kosmetikum ein oder mehrere Polysaccharide zusetzt.

Die erfindungsgemäßen Polysaccharide können aus unterschiedlichen Stoffgruppen gewählt werden.

So ist eine erfindungsgemäß vorteilhafte Ausführungsform der vorliegenden Erfindung dadurch gekennzeichnet, dass die verwendeten Polysaccharide ausgewählt werden aus der Gruppe der Gumen.

In einem solchen Fall ist es erfindungsgemäß bevorzugt, wenn die verwendeten Polysaccharide ausgewählt werden aus der Gruppe der Verbindungen Welan Gum, Sclerotium Gum und Cellulose Gum.

Die erfindungsgemäß vorteilhaften Polysaccharide können jedoch auch ausgewählt werden aus der Gruppe der Verbindungen Alginate (insbesondere Sodium Alginate) und Carboxymethylcellulose.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Für die erfindungsgemäß vorteilhaften Polysaccharide gelten für die einzelnen Stoffe die folgenden Einsatzkonzentrationen als erfindungsgemäß bevorzugt:
Welan Gum wird erfindungsgemäß bevorzugt in einer Konzentration von 0,01 bis 1,5 Gewichts-%, bezogen auf das Gesamtgewicht der Zusammensetzung eingesetzt. Als Welan Gum kann beispielsweise der Rohstoff Collstab W-100 der Firma Colltec vorteilhaft eingesetzt werden.
Sclerotium Gum wird erfindungsgemäß bevorzugt in einer Konzentration von 0,01 bis 1,5 Gewichts-%, bezogen auf das Gesamtgewicht der Zusammensetzung eingesetzt. Als Sclerotium Gum kann beispielsweise der Rohstoff Actigum CS 11der Firma Cargill vorteilhaft eingesetzt werden.

Cellulose Gum wird erfindungsgemäß bevorzugt in einer Konzentration von 0,01 bis 1,5 Gewichts-%, bezogen auf das Gesamtgewicht der Zusammensetzung eingesetzt. Als Cellulose Gum kann beispielsweise der Rohstoff Blanose Cellulose Gum der Firma Ashland vorteilhaft eingesetzt werden.

Sodium Alginate wird erfindungsgemäß bevorzugt in einer Konzentration von 0,01 bis 1,5 Gewichts-%, bezogen auf das Gesamtgewicht der Zusammensetzung eingesetzt. Als Sodium Alginate kann beispielsweise der Rohstoff Alginic Acid Sodium Salt der Firma Sigma Aldrich vorteilhaft eingesetzt werden.

Carboxymethylcellulose wird erfindungsgemäß bevorzugt in einer Konzentration von 0,01 bis 2 Gewichts-%, bezogen auf das Gesamtgewicht der Zusammensetzung eingesetzt. Als Carboxymethylcellulose kann beispielsweise der Rohstoff Aqualon CMC der Firma Ashland vorteilhaft eingesetzt werden.

Erfindungsgemäß vorteilhaft kann die Zubereitung 4-Hydroxyacetophenon enthalten. Es ist für die erfindungsgemäße Verwendung dann erfindungsgemäß vorteilhaft, wenn die kosmetische Zubereitung bzw. das Kosmetikum von 0,01 bis 2 Gewichts-% 4-Hydroxyacetophenon, bezogen auf das Gesamtgewicht der Zubereitung, enthält.

Es ist für die erfindungsgemäße Verwendung erfindungsgemäß bevorzugt, wenn die kosmetische Zubereitung bzw. das Kosmetikum von 0,1 bis 1 Gewichts-% 4-Hydroxyacetophenon, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung in Form einer Öl-in -Wasser-Emulsion (O/W-Emulsion) vorliegt.
In einem solchen Fall sind die erfindungsgemäß bevorzugten Ausführungsformen dadurch gekennzeichnet, dass die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat, Stearinsäure, Kaliumcetylphosphat, enthalten.

Darüber hinaus kann die kosmetische Zubereitung wie ein für solche Fälle übliches Kosmetikum zusammengesetzt sein und die entsprechenden, bekannten Inhaltsstoffe enthalten.

### Vergleichsversuch/Beispielrezepturen

Mit dem folgenden Versuch konnte der erfindungsgemäße Effekt beispielhaft belegt werden: Es wurden die erfindungsgemäßen Öle im erfindungsgemäß vorteilhaften Verhältnis >3:1 zu einer Butyl Methoxydibenzoylmethane und Bis-Ethylhexyloxyphenol Methoxyphenyl Triazine enthaltenden Formulierung zugesetzt und die verfleckungsreduzierende Wirkung (Reduktion db) im Vergleich zu einer Formulierung mit einem Verhältnis < 3:1 mittels einer *in vitro* Waschmethode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; db-Wert [%]**

| **INCI** | **Bsp. 1** | **Bsp. 2** | **Bsp. 3** | **Bsp. 4** | | **Bsp. 5** | **Bsp. 6** | | **Bsp. 7** | **Bsp. 8** |
|---|---|---|---|---|---|---|---|---|---|---|
| Cetearyl Alcohol | 1,00 | 1,00 | 1,00 | 1,00 | | 1,00 | 1,00 | | 1,00 | 1,00 |
| Glyceryl Stearate SE | 1,00 | 1,00 | 1,00 | 1,00 | | 1,00 | 1,00 | | 1,00 | 1,00 |
| Myristyl Myristate | 1,00 | 1,00 | 1,00 | 1,00 | | 1,00 | 1,00 | | 1,00 | 1,00 |
| Phenoxyethanol | 0,60 | 0,60 | 0,60 | 0,60 | | 0,50 | 0,50 | | 0,50 | 0,50 |
| VP/Hexadecene Copolymer | 0,50 | 0,50 | 0,50 | 0,50 | | 0,50 | 0,50 | | 0,50 | 0,50 |
| C18-38 Alkyl Hydroxystearoyl Stearate | 0,50 | 0,50 | 0,50 | 0,50 | | 0,50 | 0,50 | | 0,50 | 0,50 |
| Tocopheryl Acetate | 0,06 | 0,06 | 0,06 | 0,06 | | 0,06 | 0,06 | | 0,06 | 0,06 |
| Sodium Cetearyl Sulfate | 0,15 | 0,15 | 0,15 | 0,15 | | 0,15 | 0,15 | | 0,15 | 0,15 |
| Piroctone Olamine | | | | | | 0,50 | 0,50 | | | |
| Trisodium EDTA | 0,20 | 0,20 | 0,20 | 0,20 | | 0,20 | 0,20 | | 0,20 | 0,20 |
| Glycerin | 8,60 | 8,60 | 8,60 | 8,60 | | 8,60 | 8,60 | | 8,60 | 8,60 |
| Acrylates/C10-30 Alkyl Acrylate Crosspolymer | 0,05 | 0,05 | 0,05 | 0,05 | | 0,10 | 0,10 | | 0,10 | 0,10 |
| Xanthan Gum | 0,40 | 0,40 | 0,40 | 0,40 | | 0,40 | 0,40 | | 0,40 | 0,40 |
| Silica Dimethyl Silylate | 0,50 | 0,50 | 0,50 | 0,50 | | 0,50 | 0,50 | | 0,50 | 0,50 |
| Titanium Dioxide (nano) | 3,30 | 3,30 | 3,30 | 3,30 | | | | | | |
| Trimethoxycaprylylsilane | 0,20 | 0,20 | 0,20 | 0,20 | | | | | | |
| Alcohol Denat. | 4,00 | 4,00 | 4,00 | 4,00 | | 4,00 | 4,00 | | 4,00 | 4,00 |
| Methylparaben | 0,30 | 0,30 | 0,30 | 0,30 | | 0,30 | 0,30 | | 0,30 | 0,30 |
| Parfum | q.s. | q.s. | q.s. | q.s. | | q.s. | q.s. | | q.s. | q.s. |
| Sodium Hydroxide | q.s. | q.s. | q.s. | q.s. | | q.s. | q.s. | | q.s. | q.s. |
| Phenylbenzimidazole Sulfonic Acid | 1,00 | 1,00 | 1,00 | 1,00 | | 1,00 | 1,00 | | 1,00 | 1,00 |
| Panthenol | | | | | | 1,00 | 1,00 | | 1,00 | 1,00 |
| Bis-Ethylhexyloxyphenol Methoxyphenyl Triazine | 3,50 | 3,50 | 3,50 | 3,50 | | 3,50 | 3,50 | | 3,50 | 3,50 |
| Butyl Methoxydibenzoylmethane | 4,75 | 4,75 | 4,75 | 4,75 | | 4,75 | 4,75 | | 4,75 | 4,75 |
| Homosalate | 8,00 | 9,50 | 11,20 | 12,80 | | 9,50 | 9,50 | | 9,50 | 9,50 |
| Octocrylene | 8,00 | 9,50 | 11,20 | 12,80 | | 9,50 | 9,50 | | 9,50 | 9,50 |
| Ethylhexyl Salicylate | 4,00 | 4,75 | 5,60 | 6,40 | | 4,75 | 4,75 | | 4,75 | 4,75 |
| C12-15 Alkyl Benzoate | | | | | | | 2,00 | | | |
| Dibutyl Adipate | | | | | | | | | | 2,00 |
| Aqua | ad 100 | ad 100 | ad 100 | ad 100 | | ad 100 | ad 100 | | ad 100 | ad 100 |
| | | | | | | | | | | |
| Öl:kristalliner UV Filter | 2,8:1 | 3,1:1 | 3,3:1 | 3,6:1 | | 2,9:1 | 3,1:1 | | 2,9:1 | 3,1:1 |
| Gelbwert-Reduktion db [%] | Basis 1 | -9% | -45% | -56% | | Basis 2 | -16% | | Basis 3 | -16% |

**Fazit:** Die Auswaschbarkeit der durch Butyl Methoxydibenzoylmethane und Bis-Ethylhexyloxyphenol Methoxyphenyl Triazine hervorgerufenen Textilverfärbung wird durch die Erhöhung des Ölphasenanteils, oberhalb des minimalen Anteils zur Lösung der kristallinen UV-Filter, im Sonnenschutzmittel verbessert.

## Patentansprüche

1. Verwendung von bei Raumtemperatur flüssigen Ölen in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, **dadurch gekennzeichnet, dass** die Mindestmenge an bei Raumtemperatur flüssigen Ölen (Gesamtmenge) im Verhältnis zur Gesamtmenge an organischen, öllöslichen UV-A Filtern und organischen, öllöslichen Breitbandfiltern, bezogen auf das Gewicht Faktor 3 beträgt.

2. Verwendung nach Anspruch 1, **dadurch gekennzeichnet, dass** es sich bei den organischen, öllöslichen UV-A Filtern und/oder organischen, öllöslichen Breitbandfiltern um wie 4-(tert.-Butyl)-4'-methoxydibenzoylmethan, (2-[-4-(Diethylamino)-2-hydroxybenzoyl] Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) handelt.

3. Verwendung nach einem der vorhergehenden Ansprüche, **dadurch gekennzeichnet, dass** es sich bei den bei Raumtemperatur flüssigen Ölen um n-Dibutyladipat, C12-15 Alkyl Benzoate, Butylene Glycol Dicaprylate/Dicaprate, Ethylhexylsalicylat, (Octylsalicylate), Homomenthylsalicylat (Homosalate), Ethylhexyl-2-cyano-3,3-di-phenylacrylat (Octocrylen) und deren Mischungen handelt.

## Claims

1. Use of oils that are liquid at room temperature in cosmetic preparations comprising organic, oil-soluble UVA filters and/or broadband filters, for increasing the washability of these UV filters from textiles contaminated with the preparation, **characterized in that** the minimum amount of oils that are liquid at room temperature (total amount) in the ratio to the total amount of organic, oil-soluble UVA filters and organic, oil-soluble broadband filters, is based on the weight factor 3.

2. Use according to Claim 1, **characterized in that** the organic, oil-soluble UVA filters and/or organic, oil-soluble broadband filters are such as 4-(tert-butyl)-4'-methoxydibenzoylmethane, hexyl (2-[4-(diethylamino)-2-hydroxybenzoyl]benzoate (INCI: Diethylamino Hydroxybenzoyl Hexyl Benzoate) and 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

3. Use according to either of the preceding claims, **characterized in that** the oils that are liquid at room temperature are n-dibutyl adipate, C12-15 alkyl benzoate, butylene glycol dicaprylate/dicaprate, ethylhexyl salicylate (octyl salicylate), homomenthyl salicylate (homosalate), ethylhexyl 2-cyano-3,3-diphenylacrylate (octocrylene) or mixtures thereof.

## Revendications

1. Utilisation d'huiles liquides à température ambiante dans des préparations cosmétiques contenant des filtres UV-A organiques solubles dans les huiles et/ou des filtres à bande large pour augmenter la rinçabilité de ces filtres UV à partir de textiles contaminés avec la préparation, **caractérisée en ce que** la quantité minimale d'huiles liquides à température ambiante (quantité totale) par rapport à la quantité totale de filtres UV-A organiques solubles dans les huiles et de filtres à bande large organiques solubles dans les huiles, en termes de poids, est d'un facteur de 3.

2. Utilisation selon la revendication 1, **caractérisée en ce que** les filtres UV-A organiques solubles dans les huiles et/ou les filtres à bande large organiques solubles dans les huiles sont le 4-(tert.-butyl)-4'-méthoxydibenzoylméthane, l'ester hexylique de l'acide (2-[4-(diéthylamino)-2-hydroxybenzoyl]benzoïque (INCI : Diethylamino Hydroxybenzoyl Hexyl Benzoate) et la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

3. Utilisation selon l'une quelconque des revendications précédentes, **caractérisée en ce que** les huiles liquides à température ambiante sont l'adipate de n-dibutyle, le benzoate d'alkyle en C12-15, le dicaprylate/dicaprate de butylène glycol, le salicylate d'éthylhexyle (salicylate d'octyle), le salicylate d'homomenthyle (homosalate), le 2-cyano-3,3-diphénylacrylate d'éthylhexyle (octocrylène) et leurs mélanges.
